# FASCICULE DE BREVET EUROPEEN

(11) **EP 0 564 350 B1**
(45) Date de publication et mention de la délivrance du brevet: **14.05.1997**
(21) Numéro de dépôt: 93400811.1
(22) Date de dépôt: 30.03.1993
(51) Int. Cl.: C07D 311/30, C07D 405/12, A61K 31/35

(54) **Nouvelles 3',5'-ditertbutyl-4'-hydroxy flavones, leur procédé de préparation et les compositions pharmaceutiques exerçant une activité anti-oxydante et anti-vasoconstrictrice**
3',5'-Ditertbutyl-4'-Hydroxyflavone, Verfahren zu ihrer Herstellung und pharmazeutische Zusammensetzung mit Antioxidations- und Antivasokonstriktionsaktivität
3',5'-ditertbutyl-4'-hydroxyflavones, method for their preparation and pharmaceutical compositions with an antioxidant and antivasoconstricting activity

(30) Priorité: 31.03.1992 FR 9203861
(43) Date de publication de la demande: 06.10.1993
(73) Titulaire: ADIR ET COMPAGNIE, 92415 Courbevoie Cédex (FR)
(72) Inventeur: Rolland, Yves, F-92170 Vanves (FR); Lewin, Guy, F-92500 Rueil Malmaison (FR); Vilaine, Jean-Paul, F-92290 Chatenay Malabry (FR); Lenaers, Albert, F-78510 Triel Sur Seine (FR); Thollon, Catherine, F-75011 Paris (FR)

(56) Documents cités:
- CH-A- 406 242
- FR-A- 1 247 909
- FR-A- 2 100 766
- FR-M- 478

## Description

La présente invention a pour objet des 3'-5'-ditert.butyl-4'-hydroxy flavones, leur procédé de préparation et les compositions pharmaceutiques les renfermant.

Elle concerne particulièrement des 3',5'-ditert.butyl-4'-hydroxy flavones de formule générale (I): 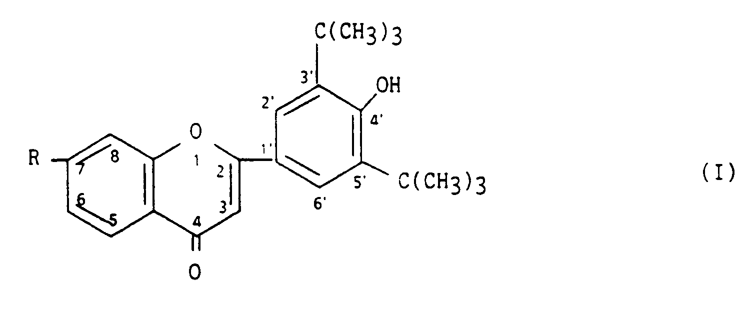 dans laquelle :
R représente :
- un atome d'hydrogène ou
- un radical OR' dans lequel R' représente :
   a) un atome d'hydrogène
   b) un radical alkyle contenant de 1 à 10 atomes de carbone en chaîne droite ou ramifiée éventuellement substituée par un ou plusieurs substituants choisis parmi le groupe formé de :
      α) un radical phényle et des radicaux hétérocycliques aromatiques mono- et bi- cycliques, chacun éventuellement substitué par un ou plusieurs atomes d'halogène, tel que chlore, brome ou fluor, et radicaux trifluorométhyle, hydroxy, alkyle et alkoxy ayant chacun de 1 à 5 atomes de carbone en chaîne droite ou ramifiée,
      β) un radical carboxy,
      γ) un radical alkoxycarbonyle dans lequel le groupe alkoxy renferme de 1 à 5 atomes de carbone en chaîne droite ou ramifiée,
      δ) un radical aminocarbonyle de formule : 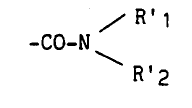 dans laquelle R'₁ et R'₂, identiques ou différents, représentent chacun : un atome d'hydrogène, un radical alkyle ayant de 1 à 5 atomes de carbone en chaîne droite ou ramifiée ou R'1 et R'2 forment ensemble avec l'atome d'azote auquel ils sont liés un radical hétérocyclique renfermant éventuellement un deuxième hétéroatome choisi parmi les atomes d'oxygène, d'azote et de soufre, lequel radical hétérocyclique peut être substitué par un radical alkyle ayant de 1 à 5 atomes de carbone en chaîne droite ou ramifiée ou un radical aralkyle, tel que par exemple un radical benzyle, dont la partie aryle est éventuellement substituée par un ou plusieurs radicaux alkyle ou alkoxy ayant chacun de 1 à 5 atomes de carbone en chaîne droite ou ramifiée,
      ε) un radical amino de formule 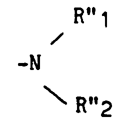 dans laquelle R''₁ et R''₂, identiques ou différents, représentent chacun :
         - un atome d'hydrogène, un radical alkyle ou hydroxyalkyle ayant chacun de 1 à 5 atomes de carbone en chaîne droite ou ramifiée, ou
         - R''₁ et R''₂ forment ensemble avec l'atome d'azote auquel ils sont liés un hétérocycle comportant éventuellement un autre hétéroatome : oxygène, azote ou soufre,
      ζ) un radical -OR'' dans lequel R'' représente un atome d'hydrogène, un radical alkyle ayant de 1 à 5 atomes de carbone, en chaîne droite ou ramifiée ou un groupe -COA dans lequel : A représente un radical alkyle ayant de 1 à 5 atomes de carbone en chaîne droite ou ramifiée, ou un radical 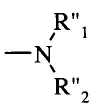 dans lequel R''₁ et R''₂ sont tels que précédemment définis, et
      η) le radical SO₃H et le radical SO₃M dans lequel M représente un métal alcalin ;
   c) un radical acyle de formule : -COR''' dans laquelle R''' représente :
      - un radical alkyle ayant de 1 à 10 atomes de carbone en chaîne droite ou ramifiée,
      - un radical aralkyle dont la partie aryle est éventuellement substituée par un ou plusieurs substituants choisis parmi les atomes d'halogène et les radicaux hydroxy, alkyle et alkoxy ayant chacun de 1 à 5 atomes de carbone en chaîne droite ou ramifiée, ou
      - un radical aryle éventuellement substitué comme la partie aryle du radical aralkyle ci-dessus défini,
         ou
   d) un radical tosyle,
leurs stéréo-isomères ainsi que leurs éventuels sels d'addition à un acide ou à base pharmaceutiquement acceptable.

Parmi les composés connus comme agents anti-inflammatoires, M. Baraldi et al. (71th Int. Conf. Prostagland. Relat. Compounds, May 28, (1990), Firenze, 222 sqq) décrit des dérivés benzo-hétérocycliques substitués par un radical 3,5-ditert-butyl-4-hydroxyphényle. Un dérivé benzo-hétérocyclique cité en exemple est la 5,5-dioxo-benzothiadiazine. Des benzohétérocycles oxygénés sont décrits dans GB-A-1250388 et par J. Adams (J. Org. Chem., (1967), 32, 3992-3998) qui concernent des 3',5'-ditert-butyl-4'-hydroxy flavanones et chalcones, diversement substituées.
Les brevets FR-A-1 247 909 et CH-A-406 242 décrivent comme composés les plus proches de l'invention des flavones non substitués sur le radical phényle et possédant une activité coronarodilatatrice. Le brevet de médicament FR-M-478 décrit quant à lui des flavones sans substitution sur le noyau phényle et utiles pour le traitement de l'asthme et de l'angine de poitrine. Le brevet FR-A-2 100 766 décrit également des flavones à noyau phényle non substitué et possédant une activité anti-athérosclérotique.

Les flavones formant le sujet de l'invention sont donc nouvelles et trouvent une application originale par leurs propriétés pharmaceutiques.

La présente invention a également pour objet le procédé de préparation des dérivés de formule générale (I), caractérisé en ce que :
A) l'on fait réagir le dérivé de formule générale (II) : 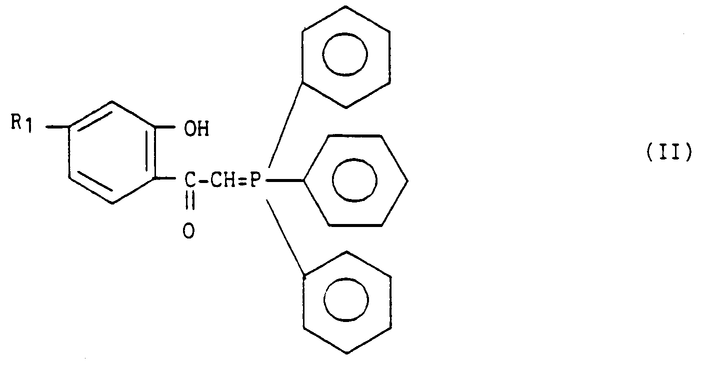 dans laquelle R₁ représente :
   - un atome d'hydrogène ou
   - un radical hydroxy,
   avec un dérivé halogéné de formule générale (III) : 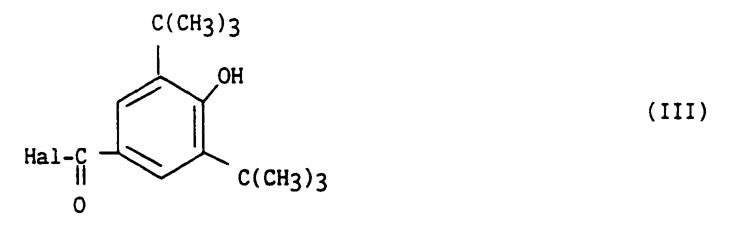 dans laquelle Hal est un atome d'halogène,
   pour donner, selon que R₁ représente un atome d'hydrogène ou un radical hydroxy,
   - soit le dérivé de formule (Ia) : 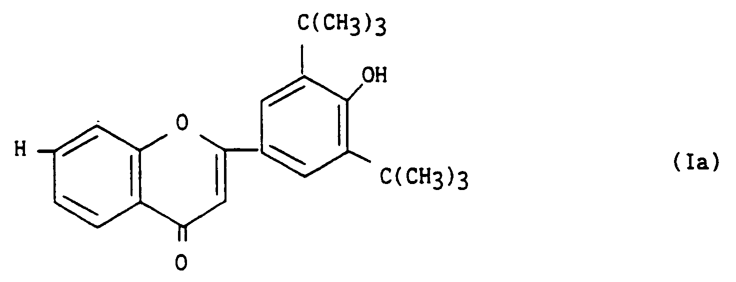
   - soit, après traitement alcalin, le dérivé de formule(Ib) : 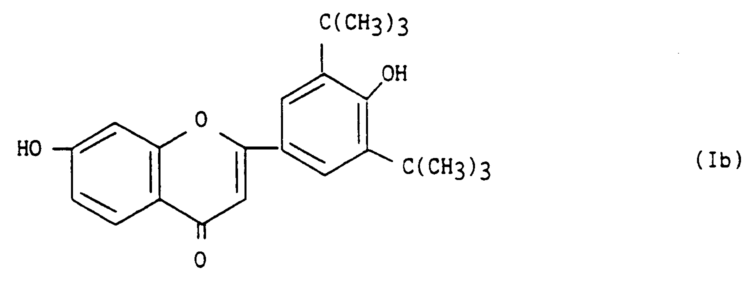 et
B) l'on traite le dérivé (Ib) ainsi obtenu par un dérivé de formule générale (IV) :

   X-R'_{A} (IV)

   dans laquelle R'_{A} a la signification de R', à l'exception d'un atome d'hydrogène et X représente un groupe partant approprié, tel que par exemple un atome d'halogène, un radical tosyle ou un radical sulfate,
   pour donner les dérivés de formule générale (Ic) : 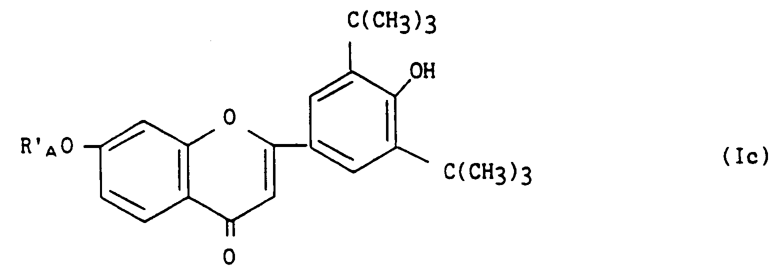 dans laquelle R'_{A} a la signification précédemment définie,

l'ensemble des dérivés de formule (Ia), (Ib) et (Ic) formant l'ensemble des dérivés de formule générale (I), que l'on purifie selon une technique classique de purification et que l'on transforme, le cas échéant, en leurs sels d'addition à un acide ou à une base pharmaceutiquement acceptable.

La réaction des dérivés (II) et (III) s'effectue de façon particulièrement adéquate en opérant dans un solvant approprié comme, par exemple la pyridine ou un mélange toluène pyridine, en chauffant à reflux pendant environ trois heures.

La réaction des dérivés (Ib) et (IV) est réalisée dans un milieu approprié tel que le diméthylformamide en présence d'hydrogénocarbonate de potassium.

Le dérivé de formule (Ib) a également été préparé en traitant par la soude le dérivé de formule générale (Ic), dans laquelle R'_{A} est limité a un radical acyle de formule : -COR''' dans laquelle R''' représente :
- un radical alkyle ayant de 1 à 10 atomes de carbone en chaîne droite ou ramifiée,
- un radical aralkyle dont la partie aryle est éventuellement substituée par un ou plusieurs substituants choisis parmi les atomes d'halogène et les radicaux hydroxy, alkyl et alkoxy ayant chacun de 1 à 5 atomes de carbone en chaîne droite ou ramifiée, ou
- un radical aryle éventuellement substitué comme la partie aryle du radical aralkyle ci-dessus défini.

Les composés de formule générale (Ic) dans laquelle R'_{A} renferme un groupement acide carboxylique terminal ont également été préparés par saponification de leurs esters correspondants. Le sel de cet acide carboxylique a été préparé de manière aisée par simple salification par l'hydrogénocarbonate de sodium en présence de tétrahydrofurane et d'eau.

Les composés de formule générale (Ic) dans laquelle R'_{A} renferme une fonction ester d'alkyle supérieur (chaîne alkyle comportant au moins 4 atomes de carbone), ont également été préparés par transestérification, à reflux pendant 5 heures en présence d'acide tosylique.

Un autre moyen utilisé pour préparer le dérivé de formule générale (Ic), dans laquelle R'_{A} comporte une fonction acide sulfonique, est la réaction du composé de formule générale (Ib) avec la 1,3-propane sultone (V) : 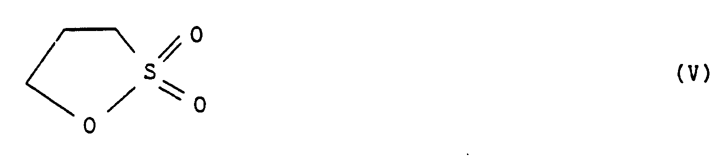 en chauffant 3 heures à environ 110 °C en présence d'hydrogénocarbonate de potassium dans le diméthylformamide.

Les nouveaux composés de la présente invention possèdent des propriétés pharmacologiques et thérapeutiques intéressantes. En particulier, pour ces dérivés, a été mise en évidence in vitro leur capacité d'une part à protéger les LDL humaines (Low Density Lipoproteins : lipoprotéines de faible densité assurant le transport du cholestérol) vis à vis des modifications oxydatives induites par le cuivre et par les cellules endothéliales ; et d'autre part à induire une relaxation vasculaire, en particulier coronaire.

Les modifications oxydatives des LDL apparaissent actuellement constituer un mécanisme important de la formation et de l'extension des lésions vasculaires athéromateuses.

D'une façon générale, les dérivés de la présente invention trouvent leur utilisation dans la protection vis à vis de troubles vasculo-tissulaires en relation avec l'oxydation de structures biologiques. Ils sont notamment utilisables comme médicament dans le traitement :
- des dyslipidémies pour prévenir leurs complications notamment vasculaires,
- de l'athérosclérose avec ses différentes localisations vasculaires périphériques, coronaires, cérébrales,
- mais aussi des pathologies dans lesquelles une peroxydation lipidique membranaire joue un rôle initiateur et/ou aggravant telles les cardiopathies ischémiques, la reperfusion d'organes, y compris transplantés, les pathologies ischémiques, traumatiques ou dégénératives du système nerveux central ou périphérique, les maladies inflammatoires aigües ou chroniques et les maladies auto-immunes.

De plus, les propriétés antispastiques des gros troncs vasculaires et les propriétés vasodilatatrices des produits de l'invention, s'avèrent intéressantes dans le cadre des pathologies vasculaires coronaires, cérébrales et périphériques tant pour leurs manifestations symptomatiques que pour la prévention de l'extension et des complications des lésions vasculaires athéroscléreuses.

Les composés de l'invention sont donc utilisables dans la protection vasculaire, dans le domaine artériel, microcirculatoire et veino-veinulaire, et en particulier pour l'insuffisance veineuse chronique, fonctionnelle et organique.

L'invention s'étend aussi aux compositions pharmaceutiques renfermant comme principe actif au moins un composé de formule générale (I) en association avec un ou plusieurs excipients inertes, non toxiques et appropriés.

Les compositions pharmaceutiques ainsi obtenues pourront être présentées sous diverses formes, les plus avantageuses étant les comprimés, dragées, gélules, suppositoires, solutions injectables ou buvables. La voie d'administration sera, selon le cas, orale, rectale ou parentérale.

La posologie utile est adaptable selon la nature et la sévérité de l'affection, la voie d'administration ainsi que selon l'âge et le poids du patient. De manière générale, la posologie unitaire s'échelonnera de 25 mg à 1 g par jour en une ou deux prises.

Les exemples suivants illustrent la présente invention sans toutefois la limiter en aucune façon.

Les produits de départ sont connus ou sont préparés selon des modes opératoires classiques, à partir de matières premières connues.

Les points de fusion (θ_{f} °C) des composés de l'invention sont déterminés par la méthode micro-Kofler et indiqués dans le tableau des produits exemplifiés.

### Exemple 1 :

### 3',5'-ditert-butyl-7,4'-dihydroxy flavone.

### a) Préparation du chlorure de 3,5-ditert-butyl-4-hydroxy benzoyle.

A 70 ml de chlorure de thionyle, refroidis par de la glace, sont ajoutés par portions 27 g (108,1 mM) d'acide 3,5-ditert-butyl-4-hydroxy benzoïque. Après complète dissolution, le mélange est porté une heure à reflux.

L'excès de chlorure de thionyle est alors éliminé par distillation sous vide et le résidu sec cristallise par refroidissement.

### b) Condensation.

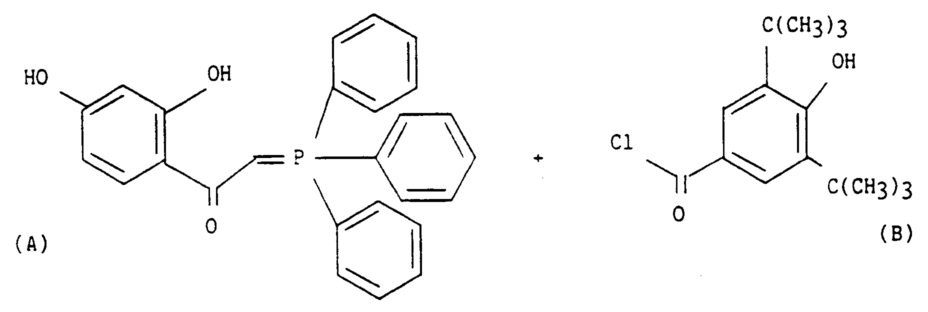

19,4 g (47 mM) de phosphorane (A) sont solubilisés dans 450 ml de pyridine anhydre à 100 °C.
En maintenant la température à 70-80 °C, le chlorure d'acyle brut (B), en solution dans 160 ml de toluène anhydre, est ajouté à la solution pyridinique pendant 10 mn environ.
La réaction est maintenue sous agitation à la même température pendant trois heures puis laissée au repos une nuit à température ambiante.
Le milieu réactionnel est filtré, versé sur de la glace et extrait par le chlorure de méthylène.
Après lavage puis séchage sur sulfate de sodium, la phase organique est évaporée sous pression réduite.
Le résidu sec est dissous dans 500 ml d'une solution de soude méthanolique 0,5 N. L'ensemble est laissé une nuit à température ambiante sous atmosphère d'azote.
Le milieu réactionnel est alors dilué à l'eau et extrait à plusieurs reprises par du cyclohexane.
La phase aqueuse est alors amenée à pH=6 par addition d'acide chlorhydrique puis extraite par le chlorure de méthylène. Après séchage sur sulfate de sodium, la phase organique filtrée est évaporée et donne 4,96 g de produit attendu sous forme de résidu sec et cristallisé.
Rendement : 29 %.

### Exemple 2 :

### 3',5'-ditert-butyl-7-n-hexyloxy-4'-hydroxy flavone.

0,183 g (0,5 mM) de composé de l'exemple 1 et 0,25 g (2,5 mM) d'hydrogénocarbonate de potassium sont agités quelques instants dans 10 ml de diméthylformamide à 110 °C sous azote jusqu'à totale dissolution. 0,7 ml (5 mM) de n-1-bromohexane sont alors ajoutés et la réaction est poursuivie dans les mêmes conditions pendant deux heures.
Le milieu réactionnel est alors repris à l'eau et extrait à l'éther éthylique.
Après traitement habituel et cristallisation du résidu sec dans un mélange chlorure de méthylène/méthanol, 0,133 g de produit attendu sont obtenus.
Rendement : 60 %

### Exemple 3 :

### 3',5'-ditert-butyl-4'-hydroxy-7-(4''-morpholinyl)éthoxy flavone.

Ce produit est obtenu selon le même mode opératoire que celui décrit dans l'exemple 2, en remplaçant le n-1-bromohexane par le 2-(4'-morpholinyl)-1-chloroéthane.

### Exemple 4 :

### 7-p-chlorobenzyloxy-3',5'-ditert-butyl-4'-hydroxy flavone.

Mode opératoire identique à celui de l'exemple 3, en utilisant le chlorure de p-chlorobenzyle.

### Exemple 5 :

### 3',5'-ditert-butyl-4'-hydroxy-7-(2''-pipéridino-2''-oxo-éthoxy)flavone.

Mode opératoire identique à celui de l'exemple 3, en utilisant le 1-pipéridino-1-oxo-2-chloroéthane.

### Exemple 6 :

### 3',5'-ditert-butyl-4'-hydroxy-7-[2''-(N-benzylpipérazino)-2''-oxo-éthoxy]flavone.

Mode opératoire identique à celui de l'exemple 3, en utilisant le 1-(N-benzylpipérazino)-1-oxo-2-chloroéthane.

### Exemple 7 :

### 3',5'-ditert-butyl-4'-hydroxy-7-(N,N-diéthyl)acétamidoxy flavone.

Mode opératoire identique à celui de l'exemple 3, en utilisant la (N,N-diéthyl)-chloroacétamide.
Rendement : 59 %.

### Exemple 8 :

### 3',5'-ditert-butyl-4'-hydroxy-7-(2''-quinolyl)méthoxy flavone.

Mode opératoire identique à celui de l'exemple 3, en utilisant le chloro-(2-quinolyl)méthane.

### Exemple 9 :

### 3',5'-ditert-butyl-4'-hydroxy-7-[2-(N,N-diméthylamino)éthoxy] flavone.

Mode opératoire identique à celui de l'exemple 3, en utilisant le 2-(N,N-diméthylamino)-1-chloroéthane.

### Exemple 10 :

### 3',5'-ditert-butyl-4'-hydroxy-7-[3''-(N,N-diméthyl)aminopropoxy]flavone.

Mode opératoire identique à celui de l'exemple 3, en utilisant le 3-(N,N-diméthyl)amino-1-chloropropane.

### Exemple 11 :

### 5-[7-(3',5'-ditert-butyl-4'-hydroxy)flavonyl]-oxy valérate d'éthyle.

Mode opératoire identique à celui de l'exemple 3, en utilisant le 1-bromovalérate d'éthyle.

### Exemple 12 :

### Acide 3-[7-(3',5'-ditert-butyl-4'-hydroxy)flavonyl]-oxy propane sulfonique.

Même mode opératoire que celui décrit dans l'exemple 3, en remplaçant le composé halogéné par la 1,3-propane sultone.

### Exemple 13 :

### 3',5'-ditert-butyl-4'-hydroxy-7-(2-hydroxy éthoxy)-flavone.

Même mode opératoire que celui décrit dans l'exemple 3, en utilisant le 2-chloro-1-hydroxy éthane.

### Exemple 14 :

### 3',5'-ditert-butyl-4'-hydroxy flavone.

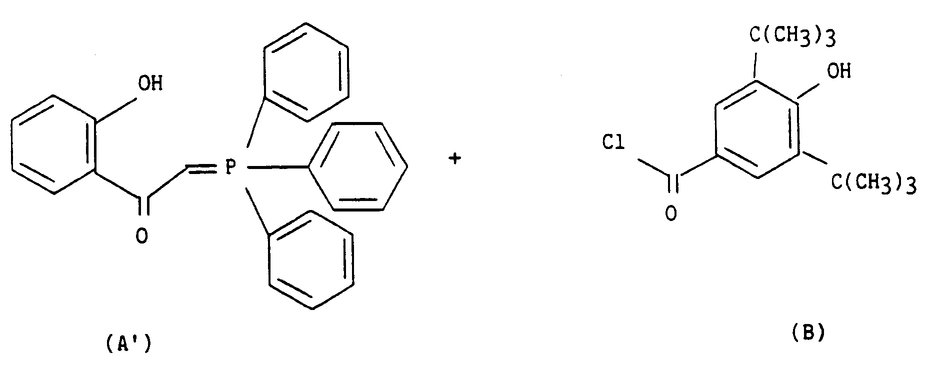

A 100 ml de mélange toluène/pyridine (95-5) et 0,806 g (1mM) de phosphorane (A') sont ajoutés 1,402 g (1,5 mM) de chlorure d'acyle (B). L'ensemble est porté à reflux pendant 3 heures.

Le milieu réactionnel est filtré, versé sur de la glace et extrait par le chlorure de méthylène.
Après lavage et séchage sur sulfate de sodium, la phase organique est évaporée sous pression réduite.
Le produit attendu est alors cristallisé dans le méthanol.

### Exemple 15 :

### 2-[7-(3',5'-ditert-butyl-4'-hydroxy)flavonyl]-oxy 2,2-diméthyl acétate d'éthyle.

Même mode opératoire que celui décrit dans l'exemple 3, en utilisant le 2-bromo-iso-butyrate d'éthyle.

### Exemple 16 :

### 2-[7-(3',5'-ditert-butyl-4'-hydroxy)flavonyl]-oxy acétate d'éthyle.

Même mode opératoire que celui décrit dans l'exemple 3, en utilisant le 2-chloroacétate d'éthyle.
Rendement : 66 %.

### Exemple 17 :

### Acide 2-[7-(3',5'-ditert-butyl-4'-hydroxy)flavonyl]-oxy acétique.

0,452 g (1 mM) de produit obtenu dans l'exemple 16 sont dissous dans 20 ml de tétrahydrofurane.
60 ml de soude aqueuse 1N sont ajoutés et la réaction est maintenue sous agitation et sous atmosphère d'azote à température ambiante pendant six heures.
Le milieu réactionnel est alors dilué à l'eau, acidifié par l'acide chlorhydrique jusqu'à pH=2 puis extrait par le chlorure de méthylène.
Le traitement habituel de la phase organique fournit un résidu sec sous forme de poudre microcristalline.
Rendement : 95 %.

### Exemple 18 :

### 2-[7-(3',5'-ditert-butyl-4'-hydroxy)flavonyl]-oxy acétate de sodium.

0,424 g (1 mM) du produit obtenu dans l'exemple 17 sont dissous dans un mélange 3/5-2/5 tétrahydrofurane-eau. 0,084 g (1 mM) d'hydrogénocarbonate de sodium sont ajoutés pour donner après traitement habituel et évaporation à sec le produit attendu.

### Exemple 19 :

### 2-[7-(3',5'-ditert-butyl-4'-hydroxy)flavonyl]-oxy acétate de pentyle.

0,271 g (0,6 mM) de produit obtenu dans l'exemple 16 et 0,030 g d'acide tosylique sont dissous dans 20 ml de n-pentanol. Le mélange est agité pendant 4 heures à 125 °C.
Le milieu réactionnel est alors dilué à l'eau et extrait par le chlorure de méthylène. Après traitement habituel de la phase organique, le résidu sec obtenu fournit, par cristallisation dans un mélange chlorure de méthylène/méthanol, le produit attendu.
Rendement : 73 %.

### Exemple 20 :

### 3',5'-ditert-butyl-4'-hydroxy-7-(3'',5''-ditert-butyl-4''-hydroxy) benzoyloxy flavone.

0,335 g (1,25 mM) de chlorure de 3,5-ditert-butyl-4-hydroxy benzoyle brut en solution dans 2 ml de toluène sont ajoutés à 0,366 g (1 mM) de produit de l'exemple 1 dissous dans 5 ml de pyridine. Après 24 heures à température ambiante, le milieu réactionnel est repris à l'eau puis extrait par le chlorure de méthylène. La phase organique est lavée puis séchée sur sulfate de sodium et évaporée à sec. Après recristallisation dans le méthanol, on obtient 0,420 g de produit attendu.
Ce composé a également été préparé directement par condensation du phosphorane (A) avec le chlorure d'acyle (B) en excès.

### Exemple 21 :

### 3',5'-ditert-butyl-4'-hydroxy-7-(2,3-dihydroxy-n-propyloxy) flavone.

Même mode opératoire que celui décrit dans l'exemple 3, l'alcool terminal du réactif étant protégé par un groupement tosyle.

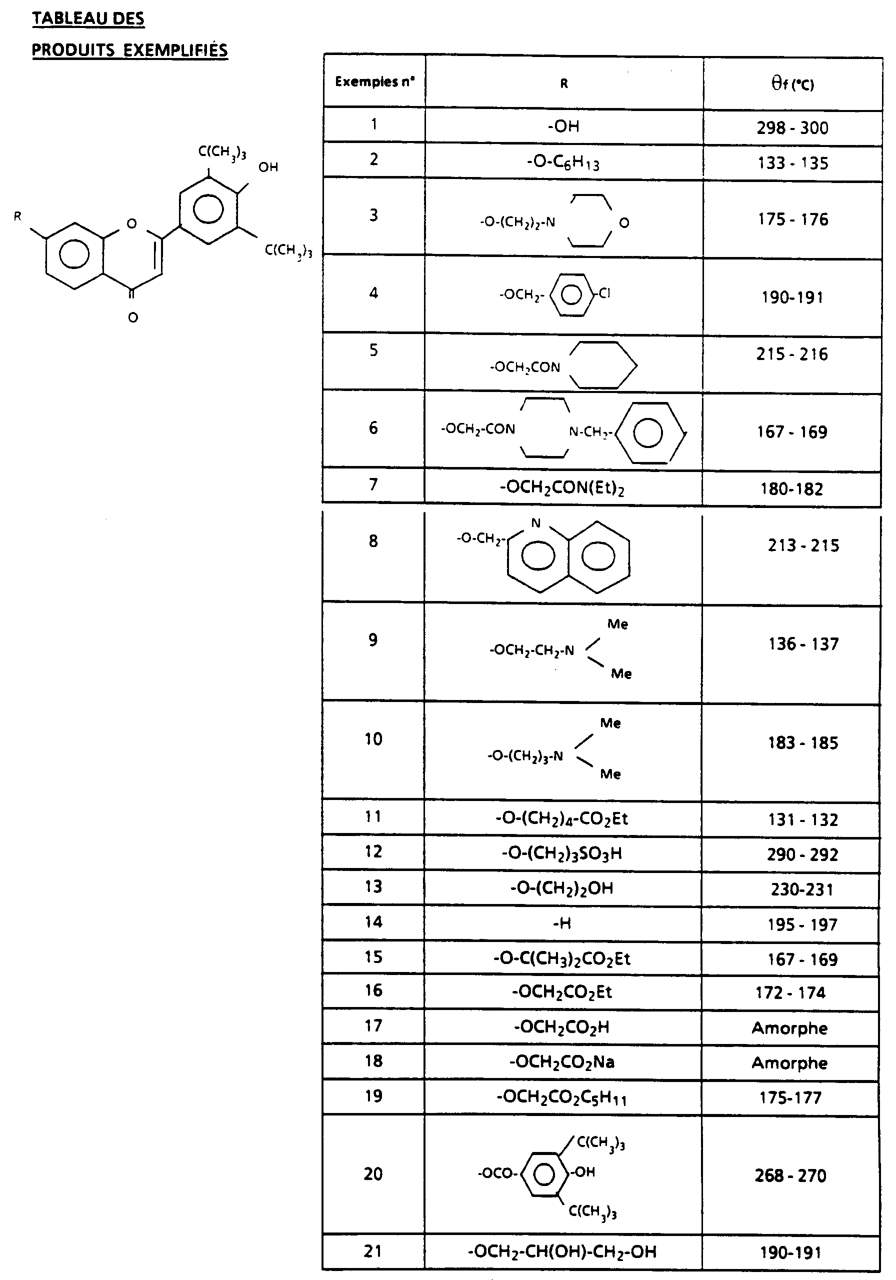

### ETUDE PHARMACOLOGIQUE

L'action des dérivés de la présente invention a été démontrée d'une part sur les LDL humaines et animales, et d'autre part sur des vaisseaux isolés.

L'activité inhibitrice des composés vis à vis de la modification oxydative des LDL, induite par le sulfate de cuivre (Exemple 21) et par des cellules endothéliales d'aorte de lapin (Exemple 22), a été démontrée in vitro. Cette activité a été comparée à celle du probucol et de la vitamine E pris comme produits de référence.

L'activité vasorelaxante a été démontrée sur des artères coronaires contractées par la prostaglandine F2α(PGF2α)(Exemple 23).

### Exemple 22 :

### Modification des LDL par le sulfate de cuivre

Des LDL humaines sont incubées 24 heures en présence de sulfate de cuivre (5 µM) et en absence ou en présence (0,1 µM à 100 µM) des composés testés.

Après incubation, la peroxydation des LDL est évaluée par électrophorèse sur gel d'agar et par formation de l'un des produits de la peroxydation lipidique : le malondialdéhyde (MDA) (cf. Parthasarathy S. et al., J. Clin. Invest., 77, (1986), 641-644).

L'activité des composés testés est évaluée par le calcul des concentrations réduisant de 50 % (IC₅₀) la production de MDA par rapport aux expériences de contrôle en absence de produit.

Les résultats de ce test d'inhibition d'oxydation des LDL par le sulfate de cuivre sont répertoriés dans le tableau ci-après.

| **COMPOSES** | **IC**_{**50**} **(µM)** |
|---|---|
| Exemple 1 | 1 |
| Exemple 2 | 5 |
| Exemple 3 | 0,8 |
| Exemple 4 | 7 |
| Exemple 5 | 3 |
| Exemple 6 | 3 |
| Exemple 7 | 3 |
| Exemple 14 | 3 |
| Exemple 15 | 3 |
| Exemple 16 | 3 |
| Exemple 17 | 3 |
| Exemple 18 | 5 |
| Exemple 20 | 30 |
| PROBUCOL | 3 |
| VITAMINE E | > 100 |

### Exemple 23 :

### Modification des LDL par les cellules endothéliales

Des LDL humaines sont incubées 24 heures en présence de cellules endothéliales d'aorte de lapin (lignée RECL B4, provenance : Pr. Steinberg, USA) et en absence ou en présence des composés testés (de 0,1 à 100 µM).

Après incubation, la peroxydation des LDL est évaluée comme dans l'exemple 21 par électrophorèse sur gel d'agar et par la formation de MDA (cf. Steinbrecher, U.P. et al., Proc. Nat. Acad, Sci. USA, 81, (1984), 3883-3887). L'activité des composés testés est évaluée par le calcul des concentrations réduisant de 50 % (IC₅₀) la production de MDA par rapport aux expériences de contrôle en absence de produit.

Les résultats de ce test d'inhibition d'oxydation des LDL par des cellules endothéliales sont regroupés ci-dessous :

| **COMPOSES** | **IC**_{**50**}**(µM)** |
|---|---|
| Exemple 14 | 2,5 |
| Exemple 15 | 0,5 |
| Exemple 16 | 0,8 |
| Exemple 17 | 0,5 |
| Exemple 20 | 0,7 |
| PROBUCOL | 4 |
| VITAMINE E | 4 |

### Exemple 24 : Etude des composés de l'invention sur des vaisseaux isolés

Des microporcs Yucatan (Ch. River) sont anesthésiés au pentobarbital. La branche circonflexe de l'artère coronaire gauche est prélevée et des anneaux proximaux d'environ 4 mm sont préparés. Les organes sont placés dans une cuve thermostatée de 20 ml contenant une solution physiologique de composition :

| | |
|---|---|
| NaCl | 118 mM |
| KCl | 4,7 mM |
| KH₂PO₄ | 1,2 mM |
| MgSO₄ | 1,2 mM |
| CaCl₂ | 2,5 mM |
| NaHCO₃ | 20 mM |
| Glucose | 11 mM |

maintenue à 37 °C, pH=7,4 et à 95 % d'oxygène et 5 % de gaz carbonique.

La tension initiale est établie progressivement jusqu'à 6 g. Après une période de stabilisation de 60 mn, les anneaux sont contractés par la PGF2α (4 µM) en présence d'indométhacine (10 µM). Une fois cette concentration stabilisée, les produits de la présente invention sont testés par addition de concentrations cumulatives du produit de l'exemple 17 et du probucol, toutes les 15 minutes, par rapport aux expériences de contrôle avec le solvant utilisé (diméthylsulfoxyde : DMSO).

Le tableau suivant donne l'évolution des tensions mesurées en pourcentage de la tension maximale observée sous PGF2α.

| | **Concentrations (µM)** | | | | |
|---|---|---|---|---|---|
| **Produits testés** | **0,1** | **0,3** | **1** | **3** | **10** |
| **Exemple 16** | 95,4 ± 2,1 | 85,0 ± 5,4 | 74,1 ± 5,7 | 37,3 ± 11,6 | 20,2 ± 10,9 |
| **DMSO** | 87,9 ± 7,3 | ― | 80,7 ± 10,5 | ― | 72,0 ± 12,1 |
| **PROBUCOL** | 93,3 ± 3,3 | 85,6 ± 4,7 | 77,3 ± 6,1 | 66,01 ± 7,9 | 49,2 ± 11,6 |

Le produit de l'exemple 16, contrairement au probucol provoque une relaxation vasculaire dépendante de la concentration permettant le calcul d'une IC₅₀ d'une valeur d'environ 2,6 µM.

L'activité de plusieurs composés de l'invention est supérieure à celle du probucol en ce qui concerne les modifications induites par le sulfate de cuivre et par les cellules endothéliales. Les composés de l'invention sont par ailleurs plus puissants que la vitamine E sur ces deux tests.

L'ensemble des résultats rapportés démontre que les composés de l'invention présentent, à des concentrations voisines, une double activité : d'une part anti-oxydante protégeant notamment les LDL vis à vis des modifications oxydatives pathogènes, d'autre part anti-vaconstrictrice notamment au niveau des artères coronaires.

Ce profil original diffère en particulier de celui du probucol et des autres antioxydants décrits et montre le grand intérêt thérapeutique des produits de l'invention.

## Revendications

1. Composés de formule générale (I) : 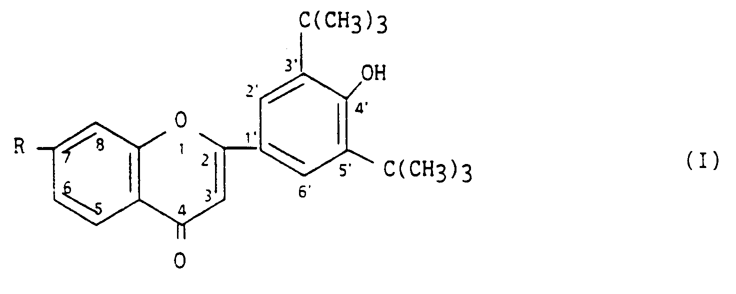 dans laquelle :
R représente :
- un atome d'hydrogène ou
- un radical OR' dans lequel R' représente :
a) un atome d'hydrogène
b) un radical alkyle contenant de 1 à 10 atomes de carbone en chaîne droite ou ramifiée éventuellement substituée par un ou plusieurs substituants choisis parmi le groupe formé de :
α) un radical phényle et des radicaux hétérocycliques aromatiques mono- et bi- cycliques, chacun éventuellement substitué par un ou plusieurs atomes d'halogène et radicaux trifluorométhyle, hydroxy, alkyle et alkoxy ayant chacun de 1 à 5 atomes de carbone en chaîne droite ou ramifiée,
β) un radical carboxy,
γ) un radical alkoxycarbonyle dans lequel le groupe alkoxy renferme de 1 à 5 atomes de carbone en chaîne droite ou ramifiée,
δ) un radical aminocarbonyle de formule : 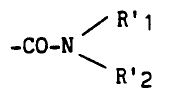 dans laquelle R'₁ et R'₂, identiques ou différents, représentent chacun : un atome d'hydrogène, un radical alkyle ayant de 1 à 5 atomes de carbone en chaîne droite ou ramifiée où R'1 et R'2 forment ensemble avec l'atome d'azote auquel ils sont liés un radical hétérocyclique renfermant éventuellement un deuxième hétéroatome choisi parmi les atomes d'oxygène, d'azote et de soufre, lequel radical hétérocyclique peut être substitué par un radical alkyle ayant de 1 à 5 atomes de carbone en chaîne droite ou ramifiée ou un radical aralkyle, dont la partie aryle est éventuellement substituée par un ou plusieurs radicaux alkyle ou alkoxy ayant chacun de 1 à 5 atomes de carbone en chaîne droite ou ramifiée,
ε) un radical amino de formule 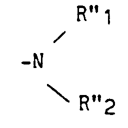 dans laquelle R''₁ et R''₂, identiques ou différents, représentent chacun :
- un atome d'hydrogène, un radical alkyle ou hydroxyalkyle ayant chacun de 1 à 5 atomes de carbone en chaîne droite ou ramifiée, ou
- R''₁ et R''₂ forment ensemble avec l'atome d'azote auquel ils sont liés un hétérocycle comportant éventuellement un autre hétéroatome : oxygène, azote ou soufre,
ζ) un radical -OR'' dans lequel R'' représente un atome d'hydrogène, un radical alkyle ayant de 1 à 5 atomes de carbone, en chaîne droite ou ramifiée ou un groupe -COA dans lequel : A représente un radical alkyle ayant de 1 à 5 atomes de carbone en chaîne droite ou ramifiée, ou un radical 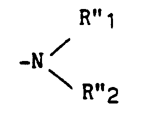 dans lequel R''₁ et R''₂ sont tels que précédemment définis, et
η) le radical SO₃H et le radical SO₃M dans lequel M représente un métal alcalin ;
c) un radical acyle de formule : -COR''' dans laquelle R''' représente :
- un radical alkyle ayant de 1 à 10 atomes de carbone en chaîne droite ou ramifiée,
- un radical aralkyle dont la partie aryle est éventuellement substituée par un ou plusieurs substituants choisis parmi les atomes d'halogène et les radicaux hydroxy, alkyle et alkoxy ayant chacun de 1 à 5 atomes de carbone en chaîne droite ou ramifiée, ou
- un radical aryle éventuellement substitué comme la partie aryle du radical aralkyle ci-dessus défini,
ou
d) un radical tosyle,
leurs stéréo-isomères ainsi que leurs éventuels sels d'addition à un acide ou à une base pharmaceutiquement acceptable.

2. Composé selon la revendication 1 qui est la 3',5'-ditert-butyl-7,4'-dihydroxy flavone.

3. Composé selon la revendication 1 qui est la 3',5'-ditert-butyl-4'-hydroxy-7-n-hexyloxy flavone.

4. Composé selon la revendication 1 qui est la 3',5'-ditert-butyl-4'-hydroxy-7-(4''-morpholinyl)éthoxy flavone.

5. Composé selon la revendication 1 qui est la 3',5'-ditert-butyl-4'-hydroxy-7-p-chlorobenzyloxy flavone.

6. Composé selon la revendication 1 qui est la 3',5'-ditert-butyl-4'-hydroxy-7-(2''-pipéridino-2''-oxo-éthoxy) flavone.

7. Composé selon la revendication 1 qui est la 3',5'-ditert-butyl-4'-hydroxy-7-[2''-(N-benzylpipérazino)-2''-oxo-éthoxy] flavone.

8. Composé selon la revendication 1 qui est la 3',5'-ditert-butyl-4'-hydroxy-7-(N,N-diéthyl)acétamidoxy flavone.

9. Composé selon la revendication 1 qui est la 3',5'-ditert-butyl-4'-hydroxy-7-(2''-quinolyl)méthoxy flavone.

10. Composé selon la revendication 1 qui est la 3',5'-ditert-butyl-4'-hydroxy-7-[2-(N,N-diméthyl)aminoéthoxy] flavone.

11. Composé selon la revendication 1 qui est la 3',5'-ditert-butyl-4'-hydroxy-7-[3''-(N,N-diméthyl)amino propoxy] flavone.

12. Composé selon la revendication 1 qui est le 5-[7-(3',5'-ditert-butyl-4'-hydroxy)flavonyl]-oxy valérate d'éthyle.

13. Composé selon la revendication 1 qui est l'acide 3-[7-(3',5'-ditert-butyl-4'-hydroxy)flavonyl]-oxy propane sulfonique.

14. Composé selon la revendication 1 qui est la 3',5'-ditert-butyl-4'-hydroxy-7-(2-hydroxy éthoxy)-flavone.

15. Composé selon la revendication 1 qui est la 3',5'-ditert-butyl-4'-hydroxy flavone.

16. Composé selon la revendication 1 qui est le 2-[7-(3',5'-ditert-butyl-4-hydroxy)flavonyl]-oxy 2,2-diméthyl acétate d'éthyle.

17. Composé selon la revendication 1 qui est le 2-[7-(3',5'-ditert-butyl-4'-hydroxy)flavonyl]-oxy acétate d'éthyle.

18. Composé selon la revendication 1 qui est l'acide 2-[7-(3',5'-ditert-butyl-4'-hydroxy)flavonyl]-oxy acétique.

19. Composé selon la revendication 1 qui est le 2-[7-(3',5'-ditert-butyl-4'-hydroxy)flavonyl]-oxy acétate de sodium.

20. Composé selon la revendication 1 qui est le 2-[7-(3',5'-ditert-butyl-4'-hydroxy)flavonyl]-oxy acétate de pentyle.

21. Composé selon la revendication 1 qui est la 3'-5'-ditert-butyl-4'-hydroxy-7-(3'',5''-ditert-butyl-4''-hydroxy) benzoyloxy flavone.

22. Procédé de préparation des composés selon les revendications 1 à 21, caractérisé en ce que :
A) l'on fait réagir le dérivé de formule générale (II) : 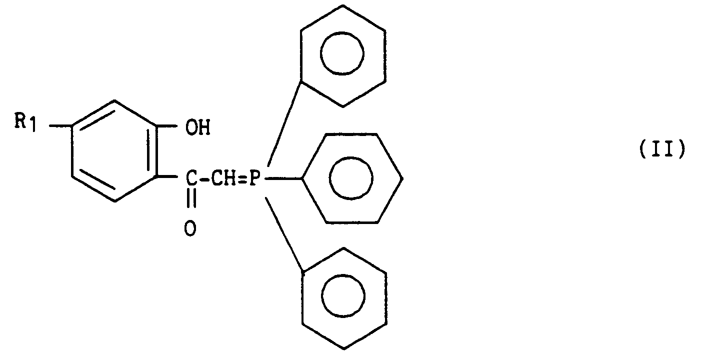 dans laquelle R₁ représente :
- un atome d'hydrogène ou
- un radical hydroxy,
avec un dérivé halogéné de formule générale (III) : 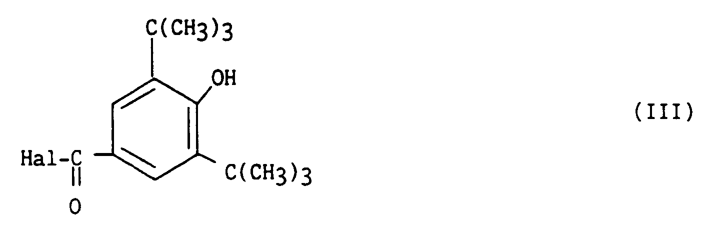 dans laquelle Hal est un atome d'halogène,
pour donner,selon que R₁ représente un atome d'hydrogène ou un radical hydroxy,
- soit le dérivé de formule (Ia) : 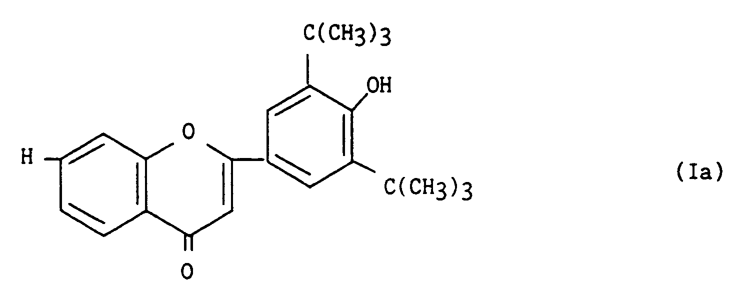
- soit, après traitement alcalin, le dérivé de formule(Ib) : 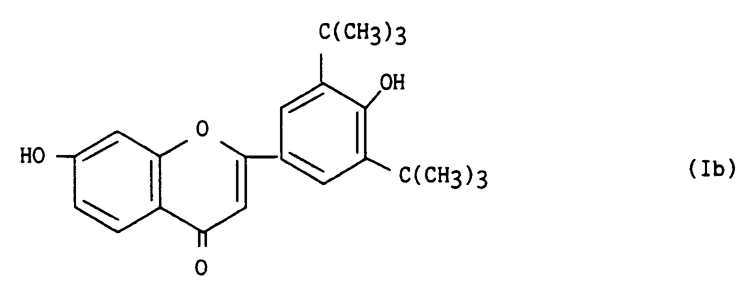 et
B) l'on traite le dérivé (Ib) ainsi obtenu par un dérivé de formule générale (IV) :
X-R'_{A} (IV)
dans laquelle R'_{A} a la signification de R' selon la revendication 1 à l'exception d'un atome d'hydrogène et X représente un groupe partant approprié,
pour donner les dérivés de formule générale (Ic) : 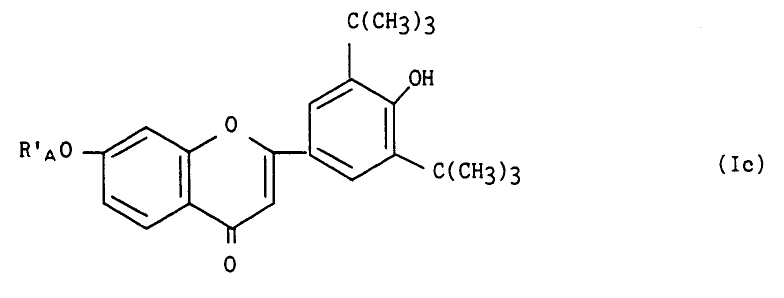 dans laquelle R'_{A} a la signification précédemment définie,
l'ensemble des dérivés de formule (Ia), (Ib) et (Ic) formant l'ensemble des dérivés de formule générale (I), que l'on purifie selon une technique classique de purification et que l'on transforme, le cas échéant, en leurs sels d'addition à un acide ou à une base pharmaceutiquement acceptable.

23. Compositions pharmaceutiques contenant comme principe actif au moins un composé selon l'une quelconque des revendications 1 à 21, seul ou en combinaison avec un ou plusieurs véhicules inertes, non toxiques, et pharmaceutiquement acceptables.

24. Compositions pharmaceutiques selon la revendication 23 excerçant une activité anti-oxydante et anti-vasoconstrictrice.

25. Compositions pharmaceutiques selon la revendication 24 utiles dans la protection vis à vis de troubles vasculo-tissulaires en relation avec l'oxydation de structures biologiques.

## Claims

1. Compounds of the general formula (I): 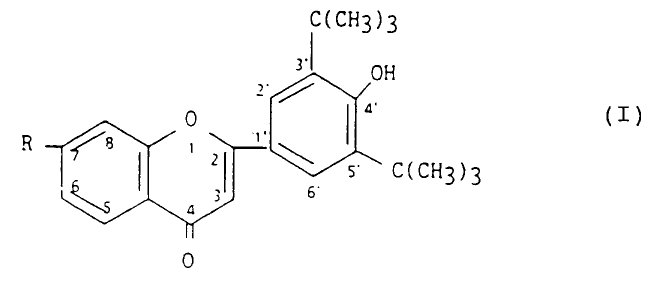 in which:
R represents:
- a hydrogen atom or
- a radical OR' in which R' represents:
a) a hydrogen atom
b) an alkyl radical containing from 1 to 10 carbon atoms in a straight or branched chain optionally substituted by one or more substituents selected from the group consisting of:
α) a phenyl radical and monocyclic and bicyclic aromatic heterocyclic radicals, all optionally substituted by one or more substituents selected from halogen atoms and trifluoromethyl and hydroxy radicals and alkyl and alkoxy radicals each having from 1 to 5 carbon atoms in a straight or branched chain,
β) a carboxy radical,
γ) an alkoxycarbonyl radical in which the alkoxy group contains from 1 to 5 carbon atoms in a straight or branched chain,
δ) an aminocarbonyl radical of the formula: 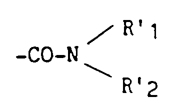 in which each of R'₁ and R'₂, which may be identical or different, represents: a hydrogen atom, or an alkyl radical having from 1 to 5 carbon atoms in a straight or branched chain, or R'₁ and R'₂ form together with the nitrogen atom to which they are bonded a heterocyclic radical optionally containing a second hetero atom selected from the atoms oxygen, nitrogen and sulphur, which heterocyclic radical may be substituted by an alkyl radical having from 1 to 5 carbon atoms in a straight or branched chain or by an aralkyl radical, the aryl moiety of which is optionally substituted by one or more alkyl or alkoxy radicals each having from 1 to 5 carbon atoms in a straight or branched chain,
ε) an amino radical of the formula 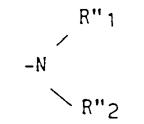 in which each of R''₁ and R''₂, which may be identical or different, represents:
- a hydrogen atom, or an alkyl or hydroxyalkyl radical each having from 1 to 5 carbon atoms in a straight or branched chain, or
- R''₁ and R''₂ form together with the nitrogen atom to which they are bonded a heterocycle optionally containing another hetero atom: oxygen, nitrogen or sulphur,
ζ) a radical -OR'' in which R'' represents a hydrogen atom, an alkyl radical having from 1 to 5 carbon atoms in a straight or branched chain, or a group -COA in which: A represents an alkyl radical having from 1 to 5 carbon atoms in a straight or branched chain, or a radical 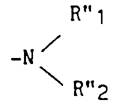 in which R''₁ and R''₂ are as defined above, and
η) the radical SO₃H and the radical SO₃M in which M represents an alkali metal;
c) an acyl radical of the formula: -COR''' in which R''' represents:
- an alkyl radical having from 1 to 10 carbon atoms in a straight or branched chain,
- an aralkyl radical, the aryl moiety of which is optionally substituted by one or more substituents selected from halogen atoms and hydroxy radicals and alkyl and alkoxy radicals each having from 1 to 5 carbon atoms in a straight or branched chain, or
- an aryl radical optionally substituted in the same manner as the aryl moiety of the aralkyl radical defined above,
or
d) a tosyl radical,
their stereoisomers and also possible addition salts thereof with a pharmaceutically acceptable acid or base.

2. Compound according to claim 1 that is 3',5'-di-tert-butyl-7,4'-dihydroxyflavone.

3. Compound according to claim 1 that is 3',5'-di-tert-butyl-4'-hydroxy-7-n-hexyloxyflavone.

4. Compound according to claim 1 that is 3',5'-di-tert-butyl-4'-hydroxy-7-(4''-morpholinylethoxy)flavone.

5. Compound according to claim 1 that is 3',5'-di-tert-butyl-4'-hydroxy-7-p-chlorobenzyloxyflavone.

6. Compound according to claim 1 that is 3',5'-di-tert-butyl-4'-hydroxy-7-(2''-piperidino-2''-oxo-ethoxy)flavone.

7. Compound according to claim 1 that is 3',5'-di-tert-butyl-4'-hydroxy-7-[2''-(N-benzylpiperazino)-2''-oxo-ethoxy]flavone.

8. Compound according to claim 1 that is 3',5'-di-tert-butyl-4'-hydroxy-7-(N,N-diethylacetamidoxy)flavone.

9. Compound according to claim 1 that is 3',5'-di-tert-butyl-4'-hydroxy-7-(2''-quinolylmethoxy)flavone.

10. Compound according to claim 1 that is 3',5'-di-tert-butyl-4'-hydroxy-7-[2-(N,N-dimethylamino)ethoxy]flavone.

11. Compound according to claim 1 that is 3',5'-di-tert-butyl-4'-hydroxy-7-[3''-(N,N-dimethylamino)propoxy]flavone.

12. Compound according to claim 1 that is ethyl 5-[7-(3',5'-di-tert-butyl-4'-hydroxy)flavonyl]oxyvalerate.

13. Compound according to claim 1 that is 3-[7-(3',5'-di-tert-butyl-4'-hydroxy)flavonyl]oxypropanesulphonic acid.

14. Compound according to claim 1 that is 3',5'-di-tert-butyl-4'-hydroxy-7-(2-hydroxyethoxy)flavone.

15. Compound according to claim 1 that is 3',5'-di-tert-butyl-4'-hydroxyflavone.

16. Compound according to claim 1 that is ethyl 2-[7-(3',5'-di-tert-butyl-4'-hydroxy)flavonyl]oxy-2,2-di-methylacetate.

17. Compound according to claim 1 that is ethyl 2-[7-(3',5'-di-tert-butyl-4'-hydroxy)flavonyl]oxyacetate.

18. Compound according to claim 1 that is 2-[7-(3',5'-di-tert-butyl-4'-hydroxy)flavonyl]oxyacetic acid.

19. Compound according to claim 1 that is sodium 2-[7-(3',5'-di-tert-butyl-4'-hydroxy)flavonyl]oxyacetate.

20. Compound according to claim 1 that is pentyl 2-[7-(3',5'-di-tert-butyl-4'-hydroxy)flavonyl]oxyacetate.

21. Compound according to claim 1 that is 3',5'-di-tert-butyl-4'-hydroxy-7-(3'',5''-di-tert-butyl-4''-hydroxy-benzoyloxy)flavone.

22. Process for the preparation of the compounds of claims 1 to 21, characterised in that :
A) a compound of the general formula (II): 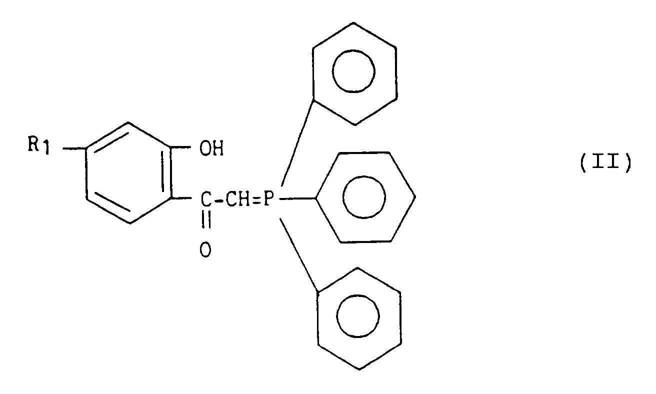 in which R₁ represents:
- a hydrogen atom or
- a hydroxy radical, is reacted with a halogenated compound of the general formula (III): 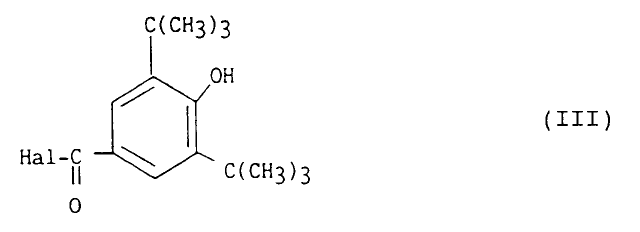 in which Hal is a halogen atom,
to yield, depending on whether R₁ represents a hydrogen atom or a hydroxy radical,
- either the compound of the formula (Ia): 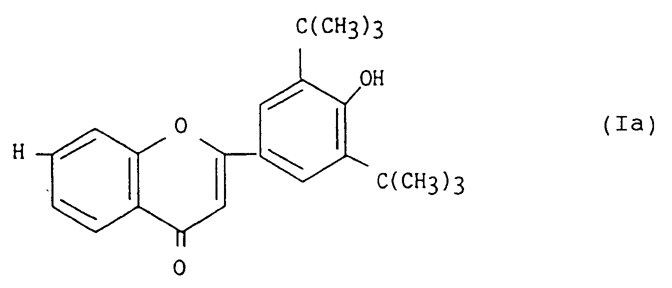
- or, after alkaline treatment, the compound of the formula (Ib): 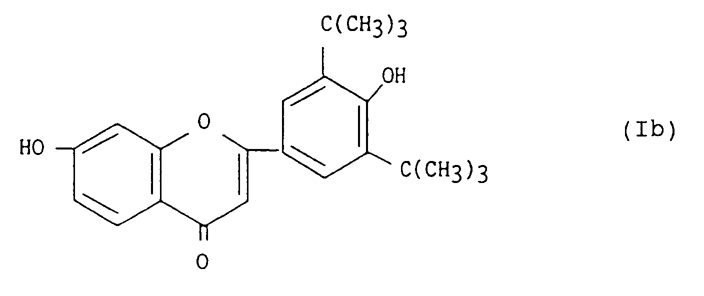 and
B) the compound (Ib) so obtained is treated with a compound of the general formula (IV):
X-R'_{A} (IV)
in which R'_{A} is as defined for R' according to claim 1, except that it cannot be a hydrogen atom, and X represents a suitable leaving group,
to yield the compounds of the general formula (Ic): 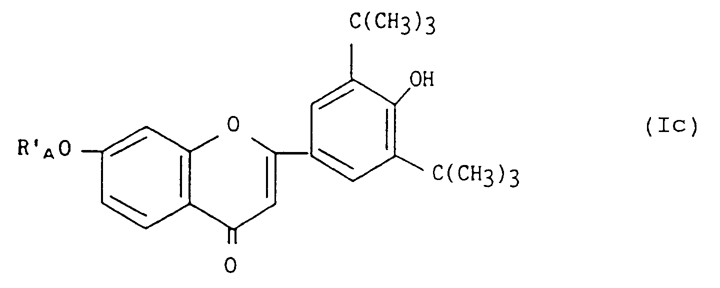 in which R'_{A} is as defined above,
all of the compounds of formulae (Ia), (Ib) and (Ic) together forming the totality of the compounds of the general formula (I), which are purified in accordance with a customary purification technique and which are converted, if desired, into their addition salts with a pharmaceutically acceptable acid or base.

23. Pharmaceutical compositions comprising as active ingredient at least one compound according to any one of claims 1 to 21, on its own or in combination with one or more pharmaceutically acceptable inert non-toxic carriers.

24. Pharmaceutical compositions according to claim 23 exercising an anti-oxidant and anti-vasoconstrictive activity.

25. Pharmaceutical compositions according to claim 24 for use in protection against vascular tissue disorders related to the oxidation of biological structures.

## Patentansprüche

1. Verbindungen der allgemeinen Formel (I): 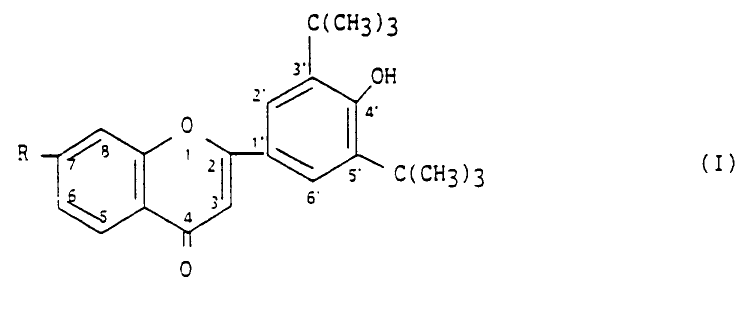 in der:
R:
- ein Wasserstoffatom oder
- eine Gruppe OR', in der R':
a) ein Wasserstoffatom,
b) eine Alkylgruppe mit 1 bis 10 Kohlenstoffatomen in gerader oder verzweigter Kette, die gegebenenfalls substituiert ist durch einen oder mehrere Substituenten ausgewählt aus der Gruppe, die gebildet wird durch:
α) eine Phenylgruppe und aromatische, mono- und bicyclische heterocyclische Gruppen, die jeweils gegebenenfalls durch eines oder mehrere Halogenatome und Trifluormethylgruppen, Hydroxylgruppen oder Alkyl- oder Alkoxygruppen mit jeweils 1 bis 5 Kohlenstoffatomen in gerader oder verzweigter Ketter substituiert sind,
β) eine Carboxygruppe,
γ) eine Alkoxycarbonylgruppe, in der die Alkoxygruppe 1 bis 5 Kohlenstoffatome in gerader oder verzweigter Kette aufweist,
δ) eine Aminocarbonylgruppe der Formel: 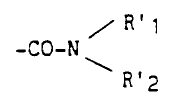 in der R'₁ und R'₂, die gleichartig oder verschieden sein können, jeweils ein Wasserstoffatom, eine Alkylgruppe mit 1 bis 5 Kohlenstoffatomen in gerader oder verzweigter Kette darstellen oder R'₁ und R'₂ gemeinsam mit dem Stickstoffatom, an das sie gebunden sind, eine heterocyclische Gruppe bilden, die gegebenenfalls ein zweites Heteroatom ausgewählt aus Sauerstoff-, Stickstoff- und Schwefelatomen aufweist, welche heterocyclische Gruppe durch eine Alkylgruppe mit 1 bis 5 Kohlenstoffatomen in gerader oder verzweigter Kette oder eine Aralkylgruppe, deren Arylrest gegebenenfalls durch eine oder mehrere Alkyl- oder Alkoxygruppen mit jeweils 1 bis 5 Kohlenstoffatomen in gerader oder verzweigter Kette substituiert ist, substituiert sein kann,
ε) eine Aminogruppe der Formel: 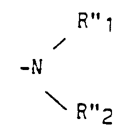 in der R''₁ und R''₂, die gleichartig oder verschieden sein können, jeweils:
- ein Wasserstoffatom, eine Alkyl- oder Hydroxyalkylgruppe mit jeweils 1 bis 5 Kohlenstoffatomen in gerader oder verzweigter Kette darstellen oder
- R''₁ und R''₂ gemeinsam mit dem Stickstoffatom, an das sie gebunden sind, einen Heterocyclus bilden, der gegebenenfalls ein weiteres Heteroatom, wie Sauerstoff, Stickstoff oder Schwefel enthalten kann,
ζ) eine Gruppe -OR'', in der R'' ein Wasserstoffatom, eine Alkylgruppe mit 1 bis 5 Kohlenstoffatomen in gerader oder verzweigter Kette oder eine Gruppe -COA darstellt, in der A eine Alkylgruppe mit 1 bis 5 Kohlenstoffatomen in gerader oder verzweigter Kette oder eine Gruppe 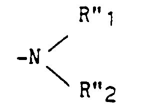 ,in der R''₁ und R''₂ die oben angegebenen Bedeutungen besitzen, darstellt und
η) die Gruppe SO₃H und die Gruppe SO₃M, in der M für ein Alkalimetall steht;
c) eine Acylgruppe der Formel -COR''', in der R''':
- eine Alkylgruppe mit 1 bis 10 Kohlenstoffatomen in gerader oder verzweigter Kette,
- eine Aralkylgruppe, deren Arylrest gegebenenfalls durch einen oder mehrere Substituenten ausgewählt aus Halogenatomen, Hydroxylgruppen und Alkyl- und Alkoxygruppen mit jeweils 1 bis 5 Kohlenstoffatomen in gerader oder verzweigter Kette substituiert ist, oder
- eine Arylgruppe, die gegebenenfalls substituiert ist, wie der Arylrest der oben definierten Aralkylgruppe, darstellt,
oder
d) eine Tosylgruppe bedeuten,
deren Stereoisomere sowie gegebenenfalls deren Additionssalze mit einer pharmazeutisch annehmbaren Säure oder Base.

2. Verbindung nach Anspruch 1, nämlich 3',5'-Ditert.-butyl-7,4'-dihydroxyflavon.

3. Verbindung nach Anspruch 1, nämlich 3',5'-Ditert.-butyl-4'-hydroxy-7-n-hexyloxyflavon.

4. Verbindung nach Anspruch 1, nämlich 3',5'-Ditert.-butyl-4'-hydroxy-7-(4''-morpholinyl)-ethoxyflavon.

5. Verbindung nach Anspruch 1, nämlich 3',5'-Ditert.-butyl-4'-hydroxy-7-p-chlorbenzyloxyflavon.

6. Verbindung nach Anspruch 1, nämlich 3',5'-Ditert.-butyl-4'-hydroxy-7-(2''-piperidino-2''-oxo-ethoxy)-flavon.

7. Verbindung nach Anspruch 1, nämlich 3',5'-Ditert.-butyl-4'-hydroxy-7-[2''-(N-benzylpiperazino)-2''-oxo-ethoxy]-flavon.

8. Verbindung nach Anspruch 1, nämlich 3',5'-Ditert.-butyl-4'-hydroxy-7-(N,N-diethyl)-acetamidoxyflavon.

9. Verbindung nach Anspruch 1, nämlich 3',5'-Ditert.-butyl-4'-hydroxy-7-(2''-chinolyl)-methoxyflavon.

10. Verbindung nach Anspruch 1, nämlich 3',5'-Ditert.-butyl-4'-hydroxy-7-[2-(N,N-dimethyl)-aminoethoxy]-flavon.

11. Verbindung nach Anspruch 1, nämlich 3',5'-Ditert.-butyl-4'-hydroxy-7-[3''-(N,N-dimethyl)-amino-propoxy]-flavon.

12. Verbindung nach Anspruch 1, nämlich 5-[7-(3',5'-Ditert.-butyl-4'-hydroxy)-flavonyl]-oxy-valeriansäureethylester.

13. Verbindung nach Anspruch 1, nämlich 3-[7-(3',5'-Ditert.-butyl-4'-hydroxy)-flavonyl]-oxy-propansulfonsäure.

14. Verbindung nach Anspruch 1, nämlich 3',5'-Ditert.-butyl-4'-hydroxy-7-(2-hydroxyethoxy)-flavon.

15. Verbindung nach Anspruch 1, nämlich 3',5'-Ditert.-butyl-4'-hydroxy-flavon.

16. Verbindung nach Anspruch 1, nämlich 2-[7-(3',5'-Ditert.-butyl-4'-hydroxy)-flavonyl]-oxy-2,2-dimethylessigsäureethylester.

17. Verbindung nach Anspruch 1, nämlich 2-[7-(3',5'-Ditert.-butyl-4'-hydroxy)-flavonyl]-oxy-essigsäureethylester.

18. Verbindung nach Anspruch 1, nämlich 2-[7-(3',5'-Ditert.-butyl-4'-hydroxy)-flavonyl]-oxyessigsäure.

19. Verbindung nach Anspruch 1, nämlich 2-[7-(3',5'-Ditert.-butyl-4'-hydroxy)-flavonyl]-oxy-essigsäurenatriumsalz.

20. Verbindung nach Anspruch 1, nämlich 2-[7-(3',5'-Ditert.-butyl-4'-hydroxy)-flavonyl]-oxy-essigsäurepentylester.

21. Verbindung nach Anspruch 1, nämlich 3',5'-Ditert.-butyl-4'-hydroxy-7-(3'',5''-ditert.-butyl-4''-hydroxy)-benzyloxyflavon.

22. Verfahren zur Herstellung der Verbindungen nach den Ansprüchen 1 bis 21, **dadurch gekennzeichnet**, daß man:
A) das Derivat der allgemeinen Formel (II): 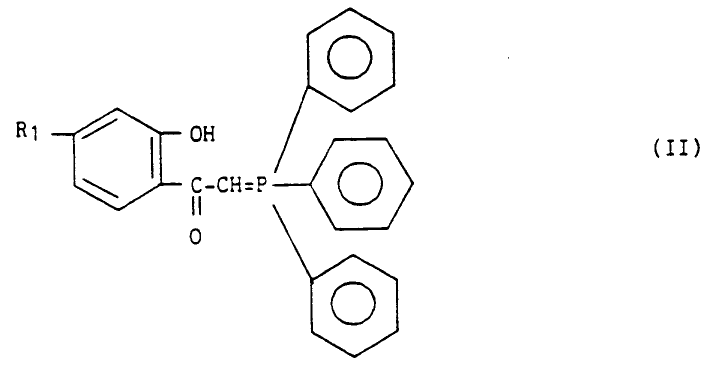 in der R₁:
- ein Wasserstoffatom oder
- eine Hydroxylgruppe
darstellt,
mit einem Halogenderivat der allgemeinen Formel (III): 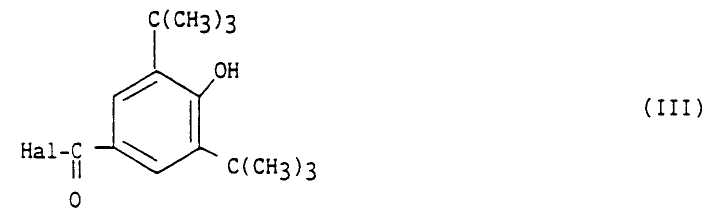 in der Hal ein Halogenatom darstellt, umsetzt,
so daß man je nachdem, ob R₁ ein Wasserstoffatom oder eine Hydroxylgruppe darstellt,
- entweder das Derivat der Formel (Ia) erhält: 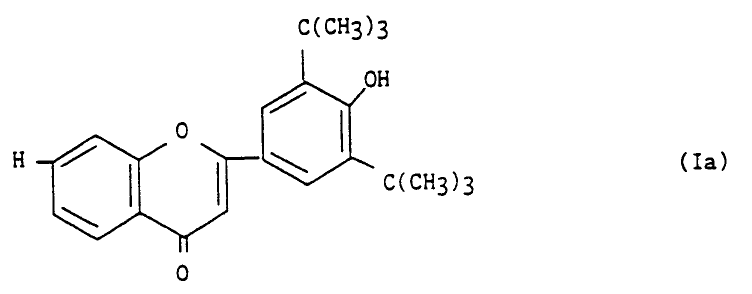
- oder nach der Behandlung mit Alkali das Derivat der Formel (Ib) erhält: 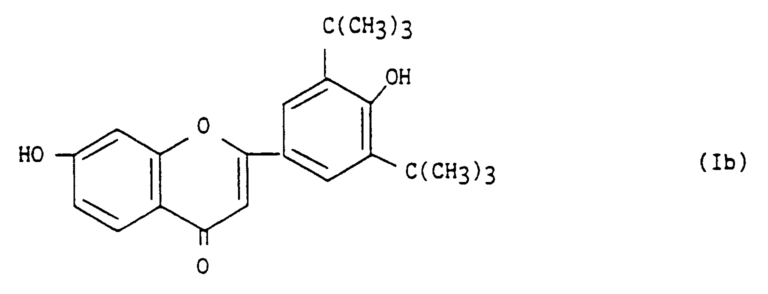 und
B) das in dieser Weise erhaltene Derivat (Ib) mit einem Derivat der allgemeinen Formel (IV):
X - R'_{A} (IV) (IV)
in der R'_{A} die in Anspruch 1 angegebenen Bedeutungen für R' besitzt mit der Ausnahme eines Wasserstoffatoms und X eine geeignete austretende Gruppe bedeutet,
behandelt zur Bildung der Derivate der allgemeinen Formel (Ic): 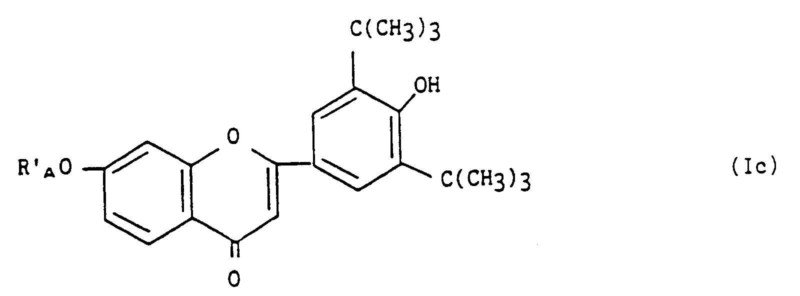 in der R'_{A} die oben angegebenen Bedeutungen besitzt,
wobei die Gesamtheit der Derivate der Formeln (Ia), (Ib) und (Ic) gemeinsam die Derivate der allgemeinen Formel (I) bilden, welche man mit Hilfe einer klassischen Reinigungsmethode reinigt und welche man gegebenenfalls mit einer pharmazeutisch annehmbaren Säure oder Base in ihre Additionssalze umwandelt.

23. Pharmazeutische Zubereitungen enthaltend als Wirkstoffmindestens eine Verbindung nach einem der Ansprüche 1 bis 21 allein oder in Kombination mit einem oder mehreren inerten, nichttoxischen und pharmazeutisch annehmbaren Trägermaterialien.

24. Pharmazeutische Zubereitungen nach Anspruch 23 mit einer antioxidierenden und einer anti-vasokontrahierenden Wirkung.

25. Pharmazeutische Zubereitungen nach Anspruch 24 zur Verwendung als Schutzgegenüber Gefäßgewebe-Störungen im Hinblick auf die Oxidation von biologischen Strukturen.
